(19) Europäisches Patentamt

European Patent Office

Office européen des brevets

(11) **EP 0 946 138 B1**

(12) **FASCICULE DE BREVET EUROPEEN**

(45) Date de publication et mention
de la délivrance du brevet:
**26.02.2003 Bulletin 2003/09**

(21) Numéro de dépôt: **97913260.2**

(22) Date de dépôt: **06.11.1997**

(51) Int Cl.⁷: **A61K 7/48**, A61K 7/06,
A61K 35/78

(86) Numéro de dépôt international:
**PCT/FR97/01988**

(87) Numéro de publication internationale:
**WO 98/019664 (14.05.1998 Gazette 1998/19)**

(54) **UTILISATION D'UN EXTRAIT DE POTENTILLA ERECTA DANS LE DOMAINE DE LA COSMETIQUE ET DE LA PHARMACIE**

VERWENDUNG EINES POTENTILLA ERECTA EXTRAKTES IM KOSMETISCHEN UND PHARMAZEUTISCHEN GEBIET

USE OF A POTENTILLA ERECTA EXTRACT IN THE COSMETIC AND PHARMACEUTICAL FIELD

(84) Etats contractants désignés:
**DE ES FR GB IT**

(30) Priorité: **07.11.1996 FR 9613585**

(43) Date de publication de la demande:
**06.10.1999 Bulletin 1999/40**

(73) Titulaire: **LVMH RECHERCHE
75008 Paris (FR)**

(72) Inventeurs:
• **BONTE, Frédéric
  F-45100 Orléans (FR)**
• **DUMAS, Marc
  F-45100 Orléans (FR)**
• **CHAUDAGNE, Catherine
  F-45530 Vitry-aux-Loges (FR)**
• **MEYBECK, Alain
  F-92400 Courbevoie (FR)**

(74) Mandataire: **Giraud, Françoise et al
Cabinet Beau de Loménie
158, rue de l'Université
75340 Paris Cedex 07 (FR)**

(56) Documents cités:
**EP-A- 0 810 222          FR-A- 2 734 478**

• **FUHRMANN: "Pflanzenextrakte für kosmetische Präparate" SEIFEN-ÖLE-FETTE-WACHSE, vol. 115, no. 2, 1989, AUGSBURG, DE, pages 38-40, XP000027104**
• **STN, Serveur de Bases de Données, XP002033781**
• **PATENT ABSTRACTS OF JAPAN vol. 95, no. 006 (C & JP 07 061916 A (SANSHO SEIYAKU CO)**
• **"Verwendung von Herbasol-Extrakten in der Kosmetik" SEIFE-ÖLE-FETTE WACHSE, vol. 107, no. 20, 1981, AUGSBURG, DE, pages 623-625, XP002033780**
• **DATABASE WPI Week 8813 Derwent Publications Ltd., London, GB; AN 88-086587 XP002033782 & HU 44 165 A (VADSZ)**
• **DATABASE WPI Week 9139 Derwent Publications Ltd., London, GB; AN 91-284717 XP002033783 & JP 03 188 008 A (SHISEIDO)**

## Description

L'invention concerne une nouvelle utilisation d'un extrait de Potentilla erecta dans le domaine de la cosmétique ou de la pharmacie, notamment en dermatologie.

La Tormentille ou Potentilla erecta Raeusch ou Potentilla tormentilla Stokes ou Neck est utilisée traditionnellement en phytomédecine comme astringent, anti-diarrhéique ou antiallergique. Voir pour cela l'ouvrage de Bisset "Herbal drugs and phytopharmaceuticals" CRC Press Ed, Boca Raton, USA, 1994, p. 499-501, ainsi que l'ouvrage intitulé : "A modem herbal" Penguin Handbooks, Harmards worth, England, 1980, p. 819-820.

La tormentille est également une plante cultivée, en particulier en Russie centrale en particulier dans l'Oural et donc facilement accessible tel que cela a été décrit par Vasfilova E.S. et Ivanova G.A. Rastit. Resur 1989, 25, 67-73.

Bos et al., dans la revue Biological and Pharmaceutical Bulletin, 1996, 19, 146-148, ont décrit récemment une activité anti-lipoperoxydante et anti-élastasique de procyanidines extraites de tormentille.

Le brevet allemand DE 3911185 décrit aussi l'utilisation d'un extrait de Potentilla erecta contre la mauvaise haleine.

La demande de brevet japonais JP 01121221 de Daiso Co décrit l'usage d'un extrait aqueux ou organique de la même plante pour traiter le diabète, cet extrait ayant des propriétés inhibitrices de l'aldose réductase.

Une publication de Selemina LV., Zozulga R.N., Yakovleva T.N. dans Rastit. Resur. 1973, 9, 409-414, décrit les propriétés antiinflammatoires et celles améliorant le renforcement de la paroi des capillaires sanguins d'un extrait de racines de tormentille.

D'autres travaux ont décrit l'utilisation de tannins de Potentilla erecta comme antiallergiques et immunostimulants (Lund et al. Deutsche Apothekr Zeitung, 1985, 125, 105-108).

Par ailleurs, on sait que le collagène de type VII, ci-après désigné par collagène VII, est le constituant prédominant des fibrilles d'ancrage, associées à la membrane basale, reliant l'épiderme au derme. Il est synthétisé par les kératinocytes de la couche basale de l'épiderme, et, en quantité moindre, par les fibroblastes du derme comme décrit par R.Burgeson dans une publication intitulée : "Type VII collagen, anchoring fibrils, and epidermolysis bullosa", J.Invest.Dermatol, (1993) 101,252-255. On pourra également se reporter à la publication de A. Koenig et al., dans J. Invest. Dermatol. (1992) 99 808-812. A ce propos, on notera que, selon des études récentes, des applications topiques d'acide rétinoïque augmentait le nombre de fibrilles d'ancrage sur des peaux ayant subi un vieillissement actinique (Woodley D.T. et al., J. Amer. Med. Assoc. (1990) 263, 3057-9). Or, l'acide rétinoïque, ou trétinoïne, est reconnu comme étant l'un des agents anti-rides les plus performants (L.H. Kligman, Cutis (1988) 41 (6) 419-20 ; J.J. Leyden et al., J. Am. Acad. Dermatol. (1989) 21 (3Pt 2) 638-44 ; J.H. Saurat, Horm. Res. (1995) 43 (1-3) 89-92)

D'après la publication de Y.Q. Chen, A.Mauviel, J.Ryynanen, S.Sollberg, J.Uitto ("Type VII collagen gene expression by human skin fibroblasts and keratinocytes in culture : Influence of donor age and cytokine responses" J. Invest.Dermatol, (1994) 102, 205-209), certaines manifestations du vieillissement cutané, telles qu'une fragilité cutanée accrue et une diminution des capacités de réparation de l'épiderme, pourraient être attribuables à une diminution de la synthèse du collagène VII chez les sujets âgés. On notera que l'expression "fragilité cutanée" recouvre en particulier l'apparition de cloques au niveau sub-basal.

Enfin, il a été décrit par M. Akiyama et al. dans J. Invest. Dermatol. (1995) 105 844-850, que le collagène, en particulier le collagène VII, jouait un rôle important au niveau du follicule pileux humain, notamment au niveau des membranes basales de la matrice (zone péripapillaire) et au niveau de la membrane basale du bulge (renflement de la partie haute du bulbe). Ces deux zones contiennent des cellules à fort potentiel mitotique, en particulier des kératinocytes donnant naissance à la tige pilaire. Or, ces auteurs ont indiqué que le collagène VII présent dans le follicule est indispensable à l'expression de cette activité mitotique.

Les auteurs de la présente invention ont maintenant mis en évidence, de façon tout à fait surprenante, qu'un extrait de Potentilla erecta possédait un effet sur la sécrétion de collagène VII par les kératinocytes humains normaux.

Cette constatation a conduit à la mise au point de nouvelles compositions cosmétiques ou pharmaceutiques, notamment dermatologiques, plus particulièrement utiles dans toutes les applications où l'on cherche à stimuler la synthèse du collagène VII.

Cette propriété s'est avérée particulièrement utile pour l'élaboration de compositions topiques cosmétiques ou dermatologiques. De telles compositions permettent, en particulier, de favoriser la cohésion entre le derme et l'épiderme chez les personnes présentant une peau atone ou relâchée. Elles se sont également avérées utiles pour les soins capillaires destinés à améliorer l'état de la chevelure. Elles permettent aussi de traiter les pathologies s'accompagnant d'une déficience de la jonction dermo-épidermique, telle que l'épidermolyse bulleuse.

Ainsi, selon l'une de ses caractéristiques essentielles, l'invention concerne l'utilisation d'un extrait de la plante Potentilla erecta comme agent cosmétique destiné à améliorer la cohésion entre le derme et l'épiderme par le renforcement de la jonction dermo-épidermique, ledit agent étant incorporé dans une composition cosmétique comprenant un véhicule cosmétiquement acceptable.

Il a été mis clairement en évidence que l'extrait incorporé dans la composition agit en stimulant la formation de collagène VII, ce qui constitue une autre caractéristique essentielle de l'invention.

Ainsi, les compositions de l'invention s'avèrent particulièrement utiles dans toutes les applications où l'on cherche à améliorer la cohésion entre le derme et l'épiderme.

La composition sera en particulier destinée à obtenir un raffermissement de la peau, à prévenir ou à retarder l'apparition des signes du vieillissement cutané, à retarder l'apparition des rides ou à diminuer leur profondeur, à améliorer l'état de la chevelure.

Il pourra, en particulier, s'agir d'un produit anti-rides, d'un produit destiné à combattre le vieillissement cutané et le relâchement de la peau ou d'une lotion de soin capillaire.

Les compositions cosmétiques de l'invention s'avèrent particulièrement intéressantes pour lutter contre le vieillissement cutané, en particulier contre le vieillissement actinique de la peau, c'est-à-dire le vieillissement induit par les rayonnements, en particulier le rayonnement solaire, tout particulièrement le rayonnement solaire ultraviolet.

D'une façon générale, les compositions cosmétiques de l'invention s'avèrent particulièrement utiles en tant que produit raffermissant de la peau destiné, en particulier, à lutter contre les peaux lâches ou atones.

Selon une autre de ses caractéristiques essentielles, l'invention concerne également l'utilisation d'un extrait de la plante Potentilla erecta pour la préparation d'une composition pharmaceutique, notamment dermatologique, destinée à traiter les pathologies liées à une déficience de la cohésion entre le derme et l'épiderme, en particulier celles liées à un affaiblissement de la jonction dermo-épidermique.

Les compositions pharmaceutiques, notamment dermatologiques de l'invention, trouveront en particulier une application dans le traitement des manifestations ou pathologies liées à une insuffisance de la formation du collagène VII.

A titre d'exemple de telles pathologies, on citera, en particulier, l'épidermolyse bulleuse.

Les compositions dermatologiques de l'invention pourront être également avantageusement utilisées comme composition cicatrisante dans le traitement des blessures, éventuellement en complément de traitements thérapeutiques locaux, en particulier pour améliorer la qualité de la peau pendant et après la cicatrisation.

Divers solvants pourront être employés pour obtenir l'extrait de la plante utilisé selon la présente invention. Toutefois, on choisira avantageusement l'eau, un alcool en $C_1$ à $C_4$, un glycol en $C_2$ à $C_6$, ou un mélange quelconque des solvants précités, en particulier un mélange hydroalcoolique ou hydroglycolique. Bien entendu, le solvant d'extraction est choisi de sorte qu'il soit liquide à température ordinaire, c'est-à-dire à 20-25°C.

L'eau sera le solvant d'extraction utilisé de préférence selon l'invention.

Parmi les alcools précités, on préfère tout particulièrement utiliser le méthanol ou l'éthanol. Parmi les glycols, on préfère utiliser l'éthylèneglycol, le propylèneglycol ou le butylèneglycol. On choisira avantageusement le butylèneglycol.

Dans les deux domaines d'applications cosmétique et pharmaceutique, notamment dermatologique, l'extrait de la plante Potentilla erecta sera avantageusement obtenu à partir du rhizome de cette plante.

Ainsi, selon une variante particulièrement avantageuse, on utilisera pour la préparation des compositions de l'invention un extrait aqueux de rhizome de Potentilla erecta.

Les compositions cosmétiques ou pharmaceutiques, notamment dermatologiques de l'invention, contiendront avantageusement de 0,0001 % à 5 %, de préférence entre 0,01 et 0,5 % en poids dudit extrait de Potentilla erecta par rapport au poids total de la composition.

Les compositions de l'invention pourront comprendre, en outre avantageusement, au moins une substance favorisant la synthèse des constituants de la matrice extracellulaire de la peau.

A titre d'exemple de telles substances, on citera les vitamines, en particulier les vitamines du groupe A et C et leurs dérivés tels que les esters, les tocophérols, les xanthines, en particulier la caféine ou la théophylline, les rétinoides, en particulier la vitamine A acide, les extraits de centella asiatica, les acides asiatiques, madécassiques et leurs dérivés glycosylés tels que l'asiaticoside ou le madécassoside, les extraits de Siegesbeckia orientalis, les extraits de Commiphora mukul et les extraits d'Eriobotrya japonica.

Par ailleurs, la composition selon l'invention peut contenir, en outre, au moins une substance choisie dans le groupe constitué par les alpha-hydroxy-acides aliphatiques en $C_3$-$C_{12}$, en particulier l'acide citrique, l'acide malique et l'acide lactique, les acides aminés, en particulier l'arginine, la citrulline et la thréonine, les céramides, les glycocéramides, les phospholipides, les agents amincissants, en particulier la forskoline, les extraits de Coleus, les extraits de Tephrosia, les agents anti-vergetures, en particulier les extraits de Marron d'Inde et l'escine, les agents protégeant ou améliorant la microcirculation, en particulier les bioflavonoïdes de Ginkgo Biloba, et les filtres solaires, en particulier les oxydes de titane, le Parsol MCX et les filtres d'origine végétale.

Dans les compositions de l'invention plus particulièrement destinées au traitement et au soin des cheveux, l'extrait de la plante Potentilla erecta sera avantageusement associé à au moins un autre principe actif choisi parmi le groupe constitué par les agents anti-pelliculaires, tels que les extraits d'Arctium lappa, le chloroxylénol, le résorcinol ou la pyrithione de zinc, les agents anti-séborrhéiques tels qu'un inhibiteur de 5α-réductase, en particulier un extrait de Pygeum africanum et les agents stimulant la microcirculation sanguine tels que la cépharanthine et le nicotinate de méthyle.

Diverses formes de formulations peuvent être réalisées. Une des formes les plus utilisées est une forme topique adaptée pour être appliquée sur le tissu cutané. Ces formulations topiques appropriées incluent, sans limitation, des émulsions, des crèmes, des laits, des baumes, des gels, des lotions ainsi que des compositions de maquillage traitantes.

L'invention concerne également selon d'autres aspects un procédé de traitement cosmétique ou pharmaceutique, notamment dermatologique, selon lequel on cherche à obtenir une amélioration de la jonction entre le derme et l'épiderme, par un renforcement de la jonction dermo-épidermique, ou une stimulation de la synthèse du collagène VII.

Cet effet est obtenu selon l'invention en appliquant sur la partie du corps à traiter d'une quantité cosmétiquement, respectivement pharmaceutiquement, efficace d'un extrait cosmétiquement, respectivement pharmaceutiquement, efficace de Potentilla erecta, ledit extrait étant incorporé dans une composition comprenant un excipient cosmétiquement, respectivement pharmaceutiquement, acceptable.

Le procédé tel que défini précédemment permettra, en particulier, d'obtenir un raffermissement de la peau, de prévenir ou retarder l'apparition des signes du vieillissement cutané, de retarder l'apparition des rides ou de diminuer leur profondeur.

Les compositions utilisées pour la mise en oeuvre de ce procédé sont celles précédemment définies.

Enfin, selon un dernier aspect, l'invention concerne également un procédé de traitement de cellules en culture, en particulier de kératinocytes humains ou de fibroblastes humains selon lequel on introduit une concentration efficace d'un extrait de la plante Potentilla erecta pour obtenir une stimulation de la synthèse du collagène VII.

Suivant une variante préférée de mise en oeuvre de ce procédé, on introduit l'extrait précité à une concentration comprise entre 0,0001 % et 1 % en poids par rapport au poids total du milieu de culture.

D'autres buts, caractéristiques et avantages de l'invention apparaissent clairement à l'homme du métier, à partir de la description explicative qui va suivre faite en référence à plusieurs exemples de réalisation donnés simplement à titre d'illustration et qui ne sauraient en aucun cas limiter la portée de l'invention. Dans les exemples qui suivent, les pourcentages sont indiqués en poids, sauf indication contraire.

EXEMPLES

Exemple 1

Préparation d'un extrait méthanolique de rhizomes de Potentilla erecta

Une partie en poids de rhizomes de la plante est extraite trois fois de suite avec 10 parties de méthanol portées à reflux pendant chaque fois 30 minutes. Tous les extraits sont regroupés après filtration.

On concentre ensuite l'extrait jusqu'à élimination totale du solvant. On récupère ainsi un produit sous forme d'extrait sec.

Exemple 2

Préparation d'un extrait de rhizomes de Potentilla erecta par extraction aqueuse

On utilise 50 g de rhizomes de la plante finement broyés et on réalise une extraction avec 500 ml d'eau portée à ébullition pendant 30 min. Après filtration sur grille de porosité 0,45 μm, le résidu solide est extrait deux fois de suite dans les mêmes conditions que la première extraction, avec 500 ml d'eau. Tous les extraits aqueux sont ensuite rassemblés puis lyophilisés. On obtient ainsi un extrait sec sous forme de poudre.

Exemple 3

Mise en évidence de la stimulation de la synthèse du collagène VII par un extrait aqueux de rhizomes de Potentilla erecta

Le test ci-dessous a été réalisé sur l'extrait aqueux sous forme de poudre lyophilisée, ci-après noté EA, obtenu selon le procédé décrit à l'exemple 2.

Les tests ont été réalisés en aveugle.

1 - Protocole du test

a) Provenance des kératinocytes :

Les cultures de kératinocytes humains normaux (KHN) sont établies à partir d'un prélèvement chirurgical de peau saine. Dans la présente étude, les tests ont été réalisés sur une souche cellulaire provenant d'un lifting d'une femme caucasienne de 58 ans.

b) Conditions de culture :

Les kératinocytes sont maintenus dans le milieu SFM (Serum Free Medium) complet (noté SFMc, GIBCO). Les cellules ont été sous-cultivées une fois à partir de la primoculture (soit un passage, noté P1).

c) Conditions de traitement :

L'ensemencement des cellules est réalisé en plaque de culture à 96 puits à raison de 30 000 KHN par puits dans le milieu SFMc. Après 24 h d'incubation nécessaire à la bonne adhérence des cellules, le milieu est remplacé par un milieu SFMc dilué à 2 %, limitant la prolifération des kératinocytes. Les solutions-mère de produit obtenu selon l'exemple 2 (noté EA dans le tableau 1) sont préparées extemporanément dans le DMSO aux concentrations de 1 - 2,5 - 5 mg/ml et introduites dans le milieu d'étude à 0,1 % V/V final (soit les concentrations testées : 1 - 2,5 - 5 µg/ml). Le témoin reçoit l'excipient du produit, soit 0,1 % V/V de DMSO. Six cultures sont réalisées pour chacune des trois concentrations et pour l'essai témoin. Le test de viabilité XTT, ainsi que l'observation microscopique des cellules, n'ont pas révélé d'effets cytotoxiques du produit aux concentrations inférieures à 10 µg/ml. (Kit XTT BOEHRINGER, Réf. 1465015).
Les cellules sont mises au contact du milieu de traitement pendant 72 h, temps requis pour une synthèse optimale de collagène VII d'après une étude cinétique préalable.
Les surnageants d'incubation sont prélevés en vue du dosage du collagène VII sécrété. Un dosage des protéines est effectué sur le tapis cellulaire restant dans les puits (méthode BCA, SIGMA), dans le but de rapporter les quantités de collagène VII sécrétées aux taux de protéines cellulaires.

d) Dosage ELISA du collagène VII :

Le protocole de dosage du collagène VII par une méthode ELISA a été adapté à partir de celui utilisé pour le dosage du collagène I (M. DUMAS, C. CHAUDAGNE, F. BONTE, A. MEYBECK : "In vitro biosynthesis of type I and III collagens by human dermal fibroblasts from donors of increasing age", Mechanisms of Ageing and Development, 73 (1994) 179-187).
Les modifications suivantes ont été apportées :

- 1er anticorps : Anticorps monoclonal de souris anti-collagène type VII humain, isotype IgG1 (Life Technologies, Réf. 12073-011, Lot FB2b01).
- 2ème anticorps : Anticorps de chèvre anti-IgG totaux de souris, couplé à la phosphatase alcaline (Interchim, Réf. 115-056-062, Lot 26793).

e) Expression des résultats et interprétation statistique :

En l'absence de collagène de type VII humain commercialement disponible pour l'établissement d'une gamme-étalon, les résultats de la sécrétion de collagène VII par les kératinocytes sont exprimés en unités de densité optique, auxquelles on soustrait l'essai témoin du dosage (notées D.O. - Blanc). Ces valeurs sont ramenées au taux de protéines cellulaires du puits correspondant (pour 72 h d'incubation).
L'activité du produit est évaluée par le pourcentage de stimulation :

[(Collagène VII des KHN traités - Collagène VII des KHN témoins) /

Collagène VII des KHN témoins) x 100.

Les résultats obtenus sur les cultures traitées (n=6) et témoins (n=6) sont comparés par le test de Student non apparié, le seuil de significativité retenu étant p<0,05.

L'activité du produit A sur le collagène VII a fait l'objet d'une confirmation, sur la même souche de KHN.

Résultats - Conclusion

Les résultats sont donnés dans le tableau 1 ci-dessous, à partir de la moyenne des mesures sur les différentes cultures :

TABLEAU 1

| COLLAGENE VII RAPPORTE AU TAUX DE PROTEINES | | | | |
|---|---|---|---|---|
| Produits | Concentrations | D.O. - Blanc collagène VII / 100 µg protéines/72 h | p (test t) par rapport au témoin | Significativité |
| Témoin SFMc 2% + DMSO 0,1% | - | 1,099+/-0,165 | | |
| EA | 2,5 µg/ml | 1,557+/-0,238 | 0,004 | S (+42%) |
| EA | 10 µg/ml | 1,490+/-0,204 | 0,004 | S (+36%) |
| N.S. : non significatif      S : significatif (p < 0,05) | | | | |

Les résultats figurant au tableau 1 montrent une stimulation importante de la synthèse du collagène VII par l'extrait aqueux de Potentilla erecta avec des concentrations de 2.5 et 10µg/ml.
Ainsi, il ressort clairement que les extraits de Potentilla erecta stimulent la formation de collagène VII. Cette protéine étant notamment le constituant principal des fibrilles d'ancrage, ces extraits peuvent donc être avantageusement utilisés comme agent pour renforcer la jonction dermo-épidermique, et améliorer ainsi la cohésion entre le derme et l'épiderme.
Par ailleurs, il est connu, notamment de la publication de M. Akiyama citée plus haut, que le collagène VII est indispensable à l'expression de l'activité mitotique des kératinocytes des follilcules pilleux humains, d'où l'intérêt des extraits de Potentilla erecta pour l'amélioration de l'état de la chevelure.

Exemple 4

Mise en évidence de la stimulation de la synthèse de collagène VII par un extrait méthanolique de rhizomes de Potentilla erecta

Cette mise en évidence est réalisée avec un extrait méthanolique, noté ci-après "EM", obtenu selon le procédé décrit à l'exemple 1.
Le protocole expérimental utilisé est exactement le même que celui décrit à l'exemple précédent relatif au test avec l'extrait aqueux, si ce n'est que les kératinocytes humains normaux (KHN) utilisés cette fois-ci sont issus d'un prélèvement (lifting) sur une femme caucasienne de 56 ans.
Les résultats obtenus ont été reportés au tableau II ci-dessous, à partir de la moyenne des mesures sur les différentes cultures.

TABLEAU II

| COLLAGENE VII RAPPORTE AU TAUX DE PROTEINES | | | | |
|---|---|---|---|---|
| Produits | Concentrations | D.O. - Blanc collagène VII / 100 µg protéines/72 h | p (test t) par rapport au témoin | Significativité |
| Témoin SFMc 2% + DMSO 0,1% | - | 1,096+/-0,146 | | |
| EM | 1 µg/ml | 1,155+/-0,069 | 0,3987 | N.S. (+5%) |
| EM | 2,5 µg/ml | 1,387+/-0,186 | 0,0138 | S (+26%) |
| EM | 5 µg/ml | 1,422+/-0,199 | 0,0100 | S (+30%) |
| N.S. : non significatif      S : significatif (p < 0,05) | | | | |

Les résultats figurant au tableau II ci-dessus, montrent que les extraits méthanoliques selon l'invention présentent, à partir d'une certaine concentration, un effet très significatif de stimulation de la formation de collagène VII.

Ces résultats confirment les conclusions du test relatif aux extraits aqueux de l'exemple précédent, à savoir que les extraits de la plante Potentilla erecta présentent un grand intérêt dans le renforcement de la jonction dermo-épidermique cutanée d'une part, et dans l'amélioration de l'état de la chevelure d'autre part.

Exemple 5

Composition de soin cosmétique contre le relâchement cutané :

Extrait méthanolique de rhizomes de Tormentille selon l'exemple 1 : 0.2 g
Extrait de Centella asiatica : 0,1 g
Vitamine C : 0,1 g
excipient émulsionné sous forme d'émulsion huile-dans-eau, parfumé et conservateurs : qsp 100 g

Cette composition combat le relâchement de la peau, et restaure sa fermeté. Elle peut être avantageusement utilisée par cures de 3 semaines en applications sur les zones du corps à traiter où la peau est relâchée.

Exemple 6

Préparation dermatologique gélifiée pour le traitement de l'épidermolyse bulleuse

Extrait méthanolique de rhizomes de Tormentille, selon l'exemple 1 : 0,5 g
carbopol 980® : 1,5 g
éthanol : 2,0 g
excipient aqueux : qsp 100 g

Cette préparation gélifiée devra être appliquée localement sur les zones à traiter en cas d'épidermolyse, 3 fois par jour pendant au moins 15 jours.

Exemple 7

Emulsion dite anti-âge

Extrait méthanolique de rhizomes de Tormentille selon l'exemple 1 : 0,2 g
Palmitate de vitamine A : 0,08 g
Ascorbyl phosphate de magnésium : 2,0 g
Céramides de blé : 0,3 g
Excipient émulsionné fluide parfumé : qsp 100 g

Cette émulsion peut être utilisée sur les zones du corps à traiter, en particulier sur le visage, de préférence tous les soirs. Cette émulsion contribue à retarder l'apparition des signes du vieillissement de la peau tels que les rides ou le relâchement cutané. Après un traitement quotidien de 6 mois environ, la peau devient plus lisse, plus souple et plus ferme. Elle reprend son éclat.

Exemple 8

Fond de teint traitant

Extrait aqueux de rhizomes de Potentilla erecta selon l'exemple 2 : 0,05 g

Poudre de Nylon SP500 : 2,0 g
Sérine : 0,1 g
Thréonine : 0,1 g
Ascorbyl phosphate de magnésium : 1,2 g
Madécassoside : 0,1 g
Excipients avec pigments et conservateurs : qsp 100 g

L'utilisation régulière de ce fond de teint contribuera à maintenir la peau en bon état et à lutter contre le vieillissement cutané.

Exemple 9

Poudre compacte pour le maquillage

Extrait méthanolique de Potentilla erecta selon l'exemple 1 : 0,08 g
Poudre Orgasol 2002 HD (Nylon) : 3,0 g
Acide lactique : 0,2 g
Excipient : qsp 100 g

Cette poudre compacte permet, tout en maquillant la peau, de retarder son vieillissement.

Exemple 10

Film occlusif pour le traitement des rides

Palmitate de vitamine A : 0,01 g
Extrait aqueux de Potentilla erecta selon l'exemple 2 : 0,05 g
Sel de magnésium de phosphate d'ascorbyle : 2,00 g
Protéines de blé : 10,00 g
Glycérol: 1,00 g
Excipients : qsp 100,00 g

Cette composition appliquée sur la peau forme, après évaporation de l'eau, un film à caractère occlusif, également désigné dans le langage professionnel par le terme "masque" ou encore par le terme anglais "patch". On applique de préférence cette composition le soir sur les zones de la peau à traiter, par exemple au dessus des pommettes sur la zone des rides en forme de "patte d'oie". Le film traitant ainsi formé est laissé en place plusieurs heures. Après 15 jours de traitement quotidien, les ridules s'estompent très nettement. Le traitement est généralement conseillé par cures de 30 jours, 3 ou 4 fois par an.

Exemple 11

Lotion capillaire pour améliorer l'état de la chevelure et combattre la formation de pellicules

Extrait méthanolique de Potentilla erecta selon l'exemple 1 : 0,1 g
Chloroxylénol : 0,05 g
Cépharantine : 0,01 g
Excipient alcoolique parfumé : qsp 100,00 g

La lotion est utilisée par applications matin et soir sur le cuir chevelu, suivies d'un léger massage. Après 8 à 15 jours de traitement, les cheveux retrouvent leur brillance et leur souplesse et les démangeaisons disparaissent. Les cures conseillées sont de 30 jours, espacées de 2 à 4 mois selon l'importance du problème capillaire à traiter.

**Revendications**

1. Utilisation d'un extrait de la plante Potentilla erecta comme agent cosmétique destiné à améliorer la cohésion entre le derme et l'épiderme par le renforcement de la jonction dermo-épidermique, ledit agent étant incorporé dans une composition cosmétique comprenant un véhicule cosmétiquement acceptable.

2. Utilisation d'un extrait de la plante Potentilla erecta comme agent cosmétique destiné à stimuler la formation de coliagène VII, ledit agent étant incorporé dans une composition cosmétique comprenant un véhicule cosmétiquement acceptable.

3. Utilisation selon la revendication 1 ou 2, **caractérisée en ce que** ladite composition est destinée à obtenir un raffermissement de la peau, à prévenir ou à retarder l'apparition des signes du vieillissement cutané, à retarder l'apparition des rides ou à diminuer leur profondeur.

4. Utilisation selon la revendication 3, **caractérisée en ce que** le vieillissement cutané est le vieillissement actinique, dû en particulier à l'action du rayonnement solaire.

5. Utilisation selon la revendication 2, **caractérisée en ce que** ledit agent est destiné à améliorer l'état de la chevelure.

6. Utilisation d'un extrait de la plante Potentilla erecta pour la préparation d'une composition pharmaceutique, notamment dermatologique, destinée à traiter les pathologies liées à une déficience de la cohésion entre le derme et l'épiderme, en particulier celles liées à un affaiblissement de la jonction dermo-épidermique.

7. Utilisation d'un extrait de la plante Potentilla erecta pour la préparation d'une composition pharmaceutique, notamment dermatologique, destinée à traiter les manifestations ou les pathologies liées à une insuffisance de la formation du collagène VII.

8. Utilisation selon la revendication 6 ou 7, **caractérisée en ce que** ladite composition est destinée au traitement de l'épidermolyse bulleuse.

9. Utilisation selon l'une des revendications 6 ou 7, **caractérisée en ce que** ladite composition est destinée au traitement des blessures en vue d'améliorer leur cicatrisation, éventuellement en complément de traitements thérapeutiques locaux, en particulier pour améliorer la qualité de la peau pendant et après la cicatrisation.

10. Utilisation selon l'une des revendications 1 à 9, **caractérisée en ce que** ledit extrait est obtenu par extraction à l'aide d'un solvant choisi dans le groupe constitué par l'eau, un alcool en $C_1$-$C_4$, un glycol en $C_2$-$C_6$ ou un mélange quelconque des solvants précités, en particulier un mélange hydroalcoolique ou hydroglycolique.

11. Utilisation selon la revendication 10, **caractérisée en ce que** ledit solvant est l'eau.

12. Utilisation selon la revendication 10, **caractérisée en ce que** ledit alcool est le méthanol ou l'éthanol.

13. Utilisation selon la revendication 10, **caractérisée en ce que** ledit glycol choisi dans le groupe constitué par l'éthylèneglycol, le propylèneglycol et le butylèneglycol.

14. Utilisation selon la revendication 13, **caractérisée en ce que** ledit extrait est obtenu par extraction avec le butylèneglycol.

15. Utilisation selon l'une des revendications 1 à 14, **caractérisée en ce que** ledit extrait est obtenu à partir des rhizomes de la plante.

16. Utilisation selon l'une des revendications 1 à 15, **caractérisée en ce que** ladite composition contient de 0,0001 % à 5 % et de préférence entre 0,01 % et 0,5 % en poids dudit extrait par rapport au poids total de ladite composition.

17. Utilisation selon l'une des revendications 1 à 16, **caractérisée en ce que** ladite composition contient en outre au moins une substance favorisant la synthèse des constituants de la matrice extracellulaire de la peau.

18. Utilisation selon la revendication 17, **caractérisée en ce que** ladite substance est choisie parmi le groupe constitué par les vitamines, en particulier les vitamines du groupe A et C et leurs dérivés tels que les esters, les tocophérols, les xanthines, en particulier la caféine ou la théophylline, les rétinoides, en particulier la vitamine A acide, les extraits de centella asiatica, les acides asiatiques, madécassiques et leurs dérivés glycosylés tels que l'asiaticoside ou le madécassoside, les extraits de Siegesbeckia orientalis, les extraits de Commiphora mukul et les extraits d'Eriobotrya japonica.

19. Utilisation selon l'une des revendications 1 à 18, **caractérisée en ce que** ladite composition contient en outre au moins une substance choisie dans le groupe constitué par les alpha-hydroxy-acides aliphatiques en $C_3$-$C_{12}$, en particulier l'acide citrique, l'acide malique et l'acide lactique, les acides aminés, en particulier l'arginine, la citrulline et la thréonine, les céramides, les glycocéramides, les phospholipides, les agents amincissants, en particulier la forskoline, les extraits de Coleus, les extraits de Tephrosia, les agents anti-vergetures, en particulier les extraits de Marron d'Inde et l'escine, les agents protégeant ou améliorant la microcirculation, en particulier les bioflavonoïdes de Ginkgo Biloba, et les filtres solaires, en particulier les oxydes de titane, le Parsol MCX et les filtres d'origine végétale.

20. Utilisation selon l'une des revendications 1 à 19, **caractérisée en ce que** ladite composition contient en outre au moins un autre principe actif choisi parmi le groupe constitué par les agents anti-pelliculaires, tels que les extraits d'Arctium lappa, le chloroxylénol, le résorcinol ou la pyrithione de zinc, les agents anti-séborrhéiques tels qu'un inhibiteur de 5$\alpha$-réductase, en particulier un extrait de Pygeum africanum, et les agents stimulant la microcirculation sanguine, tels que la cépharanthine et le nicotinate de méthyle.

21. Procédé de traitement cosmétique destiné à obtenir une amélioration de la jonction entre le derme et l'épiderme par un renforcement de la jonction dermo-épidermique ou à stimuler la formation de collagène VII, **caractérisé en ce qu'**il consiste à appliquer une quantité cosmétiquement efficace d'un extrait de la plante Potentilla erecta, ledit extrait étant contenu dans une composition comprenant un excipient cosmétiquement acceptable.

22. Procédé de traitement cosmétique selon la revendication 21, **caractérisé en ce qu'**il consiste à appliquer une quantité cosmétiquement efficace d'un extrait de la plante Potentilla erecta pour obtenir un raffermissement de la peau, prévenir ou retarder l'apparition des signes du vieillissement cutané, retarder l'apparition des rides ou diminuer leur profondeur, ledit extrait étant contenu dans une composition comprenant un excipient cosmétiquement acceptable.

**Patentansprüche**

1. Verwendung eines Extrakts der Pflanze Potentilla erecta als kosmetisches Mittel, das darauf ausgerichtet ist, den Zusammenhalt zwischen der Dermis und der Epidermis durch Verstärkung der dermal-epidermalen Verbindung zu verbessern, wobei das Mittel in einer kosmetischen Zusammensetzung, die einen kosmetisch akzeptablen Träger umfasst, eingeschlossen ist.

2. Verwendung eines Extrakts der Pflanze Potentilla erecta als kosmetisches Mittel, das darauf ausgerichtet ist, die Kollagen VII-Bildung anzuregen, wobei das Mittel in einer kosmetischen Zusammensetzung, die einen kosmetisch akzeptablen Träger umfasst, enthalten ist.

3. Verwendung gemäß Anspruch 1 oder 2, **dadurch gekennzeichnet, dass** die Zusammensetzung darauf ausgerichtet ist, eine Straffung der Haut zu erhalten, die Anzeichen der Hautalterung zu verhindern oder zu verzögern, das Auftreten von Falten zu verhindern oder ihre Tiefe zu verringern.

4. Verwendung gemäß Anspruch 3, **dadurch gekennzeichnet, dass** die Hautalterung die heliogene Alterung, vor allem aufgrund der Wirkung von Sonnenstrahlen ist.

5. Verwendung gemäß Anspruch 2, **dadurch gekennzeichnet, dass** das Mittel darauf ausgerichtet ist, den Zustand der Kopfhaut zu verbessern.

6. Verwendung eines Extrakts der Pflanze Potentilla erecta zur Herstellung einer pharmazeutischen Zusammensetzung, insbesondere einer dermatologischen, die darauf ausgerichtet ist, Krankheiten zu behandeln, die mit einem Mangel des Zusammenhalts zwischen der Dermis und der Epidermis, insbesondere solche, die mit einer Schwächung der dermal-epidermalen Verbindung zusammenhängen.

7. Verwendung eines Extrakts der Pflanze Potentilla erecta zur Herstellung einer pharmazeutischen Zusammensetzung, insbesondere einer dermatologischen, die darauf ausgerichtet ist, die Manifestierungen oder Krankheiten, die mit einem Mangel der Kollagen VII-Bildung zusammenhängen, zu behandeln.

8. Verwendung gemäß Anspruch 6 oder 7, **dadurch gekennzeichnet, dass** die Zusammensetzung auf die Behandlung von Epidermolysis bullosa ausgerichtet ist.

9. Verwendung gemäß einem der Ansprüche 6 oder 7, **dadurch gekennzeichnet, dass** die Zusammensetzung auf die Behandlung von Wunden, hinsichtlich der Verbesserung der Vernarbung ausgerichtet ist, gegebenenfalls zum Vervollständigen lokaler therapeutischer Behandlungen, insbesondere zur Verbesserung der Hautqualität während und nach der Vernarbung.

10. Verwendung gemäß einem der Ansprüche 1 bis 9, **dadurch gekennzeichnet, dass** der Extrakt durch Extraktionen mit Hilfe eines Lösungsmittels erhalten wird, das aus der Gruppe ausgewählt wird, die aus Wasser, einem $C_1$-$C_4$-Alkohol, einem $C_2$-$C_6$-Glykol oder einer Mischung der vorher erwähnten Lösungsmittel gebildet wird, insbesondere aus einer hydroalkoholischen oder hydroglykolischen Mischung.

11. Verwendung gemäß Anspruch 10, **dadurch gekennzeichnet, dass** das Lösungsmittel Wasser ist.

12. Verwendung gemäß Anspruch 10, **dadurch gekennzeichnet, dass** der Alkohol Methanol oder Ethanol ist.

13. Verwendung gemäß Anspruch 10, **dadurch gekennzeichnet, dass** der Glykol aus der Gruppe ausgewählt wird, die aus Ethylenglykol, Propylenglykol und Butylenglykol gebildet wird.

14. Verwendung gemäß Anspruch 13, **dadurch gekennzeichnet, dass** der Extrakt durch Extraktion mit Butylenglykol erhalten wird.

15. Verwendung gemäß einem der Ansprüche 1 bis 14, **dadurch gekennzeichnet, dass** der Extrakt aus den Rhizomen der Pflanze erhalten wird.

16. Verwendung gemäß einem der Ansprüche 1 bis 15, **dadurch gekennzeichnet, dass** die Zusammensetzung 0,0001 bis 5 Gew.%, und bevorzugt zwischen 0,01 und 0,5 Gew.% des Extrakts, bezogen auf das Gesamtgewicht der Zusammensetzung, enthält.

17. Verwendung gemäß einem der Ansprüche 1 bis 16, **dadurch gekennzeichnet, dass** die Zusammensetzung außerdem mindestens eine Substanz enthält, die die Synthese der Bestandteile der extrazellulären Matrix der Haut fördert.

18. Verwendung gemäß Anspruch 17, **dadurch gekennzeichnet, dass** die Substanz aus der Gruppe ausgewählt wird, die aus Vitaminen, insbesondere den Vitaminen der Gruppe A und C und ihren Derivaten, wie den Estern, den Tocopherolen, den Xanthinen, insbesondere dem Koffein oder dem Theophyllin, den Retinoiden, insbesondere der Vitamin A-Säure, den Extrakten von Centella asiatica, den asiatischen Säuren, madekassischen ihren glykosylierten Derivaten, wie beispielsweise dem Asiatikosid oder Madekassoid, den Extrakten aus Siegesbeckia orientalis, den Extrakten aus Commiphora mukul und den Extrakten aus Eriobotyra japonica gebildet wird.

19. Verwendung gemäß einem der Ansprüche 1 bis 18, **dadurch gekennzeichnet, dass** die Zusammensetzung unter anderem mindestens eine Substanz enthält, die aus der Gruppe ausgewählt wird, die aus den $C_3$-$C_{12}$-aliphatischen Hydroxysäuren, insbesondere der Zitronensäure, der Malleinsäure und der Milchsäure, den Aminosäuren, insbesondere dem Arginin, dem Citrullin und dem Threonin, den Ceramiden, den Glykoceramiden, den Phospholipiden, den Verflüssigungsmitteln, insbesondere den Buntnesselextrakten, den Tephrosia-Extrakten, den Mitteln gegen Artrophia striata et marculata, insbesondere Extrakten aus Marron d'Inde und Escin, den Stoffen zur Förderung und Verbesserung der Mikrozirkulation, insbesondere Bioflavonoiden aus Gingko Biloba und den Sonnenfiltern, insbesondere Titanoxiden, dem Parsol MCX und Filtern pflanzlichen Ursprungs gebildet wird.

20. Verwendung gemäß einem der Ansprüche 1 bis 19, **dadurch gekennzeichnet, dass** die Zusammensetzung unter anderem mindestens einen anderen Wirkstoff enthält, der aus der Gruppe ausgewählt wird, die aus den AntischuppenMitteln, wie den Extrakten aus Arctium lappa, dem Chlorxylenol, dem Resorcin und dem Zinkpyrithion, den anti-seborrhoischen Mitteln, wie beispielsweise einem $5\alpha$-Reduktaseinhibitor, insbesondere einem Extrakt aus Pygeum africanum, und den Mitteln, die die Mikrozirkulation des Bluts stimulieren, wie beispielsweise Cepharanthin und Methylnikotinat gebildet wird.

21. Verfahren zur kosmetischen Behandlung, die darauf ausgerichtet ist, eine Verbesserung der Verbindung zwischen der Dermis und der Epidermis durch eine Verstärkung der dermal-epidermalen Bindung zu erhalten oder die Bildung von Kollagen VII zu stimulieren, **dadurch gekennzeichnet, dass** es daraus besteht, eine kosmetisch wirksame Menge eines Extrakts der Pflanze Potentilla erecta aufzutragen, wobei der Extrakt in einer Zusammensetzung, die einen kosmetisch akzeptablen Arzneistoffträger enthält, enthalten ist.

22. Verfahren zur kosmetischen Behandlung gemäß Anspruch 21, **dadurch gekennzeichnet, dass** es daraus besteht, eine kosmetisch wirksame Menge eines Extrakts der Pflanze Potentilla erecta aufzutragen, um eine Straffung der Haut zu erhalten, das Auftreten von Anzeichen der Hautalterung zu verhindern oder zu verzögern, das Auftreten von Falten zu verhindern oder ihre Tiefe zu verringern, wobei der Extrakt in einer Zusammensetzung, die einen kosmetisch akzeptablen Arzneiträger umfasst, enthalten ist.

## Claims

1. Use of an extract of the plant *Potentilla erecta* as cosmetic agent intended for improving the cohesion between the dermis and the epidermis by reinforcing the dermo-epidermal junction, said agent being incorporated in a cosmetic composition comprising a cosmetically acceptable vehicle.

2. Use of an extract of the plant *Potentilla erecta* as cosmetic agent intended for stimulating the formation of collagen VII, said agent being incorporated in a cosmetic composition comprising a cosmetically acceptable vehicle.

3. Use according to claim 1 or 2, **characterised in that** said composition is intended to obtain a toning of the skin, to prevent or to delay the appearance of signs of skin ageing, to delay the appearance of wrinkles or to decrease their depth.

4. Use according to claim 3, **characterised in that** the skin ageing is actinic ageing, due in particular to solar radiation.

5. Use according to claim 2, **characterised in that** said agent is intended for improving hair condition.

6. Use of an extract of the plant *Potentilla erecta* for preparing a pharmaceutical composition, notably a dermatological composition, intended for treating pathologies linked to a deficiency in the cohesion between the dermis and the epidermis, in particular those linked to a weakening of the dermo-epidermal junction.

7. Use of an extract of the plant *Potentilla erecta* for preparing a pharmaceutical composition, notably a dermatological composition, intended for treating manifestations or pathologies linked to an insufficiency in the formation of collagen VII.

8. Use according to claim 6 or 7, **characterised in that** said composition is intended for treating epidermolysis bullosa.

9. Use according to one of claims 6 or 7, **characterised in that** said composition is intended for treating wounds with a view to improving their healing, optionally as a supplement to local therapeutic treatments, in particular for improving the quality of the skin before and after healing.

10. Use according to one of claims 1 to 9, **characterised in that** said extract is obtained by extraction with the aid of a solvent selected from the group constituted by water, a $C_1$-$C_4$ alcohol, a $C_2$-$C_6$ glycol or any mixture of the solvents mentioned above, in particular an aqueous alcohol mixture or aqueous glycol mixture.

11. Use according to claim 10, **characterised in that** said solvent is water.

12. Use according to claim 10, **characterised in that** said alcohol is methanol or ethanol.

13. Use according to claim 10, **characterised in that** said glycol is selected from the group consisting of ethylene glycol, propylene glycol and butylene glycol.

**14.** Use according to claim 13, **characterised in that** said extract is obtained by extraction with butylene glycol.

**15.** Use according to one of claims 1 to 14, **characterised in that** said extract is obtained from the plant rhizomes.

**16.** Use according to one of claims 1 to 15, **characterised in that** said composition contains 0.0001 % to 5 % and preferably between 0.01 % and 0.5 % by weight of said extract with respect to the total weight of said composition.

**17.** Use according to one of claims 1 to 16, **characterised in that** said composition further contains at least one substance which promotes the synthesis of the constituents of the extracellular matrix of the skin.

**18.** Use according to claim 17, **characterised in that** said substance is selected from the group constituted by vitamins, in particular vitamins from the A and C group and derivatives thereof such as esters, tocopherols, xanthines, in particular caffeine or theophylline, retinoids, in particular vitamin A acid, extracts of centella asiatica, asiatic acids, madecassic acids and glycosylated derivatives thereof such as asiaticoside or madecassoside, extracts of Siegesbeckia orientalis, extracts of Commiphora mukul and extracts of Eriobotrya japonica.

**19.** Use according to one of claims 1 to 18, **characterised in that** said composition further contains at least one substance selected from the group constituted by aliphatic $C_3$-$C_{12}$ alpha-hydroxy-acids, in particular citric acid, malic acid and lactic acid, amino acids, in particular arginine, citrulline and threonine, ceramides, glycoceramides, phospholipids, slimming agents, in particular forskolin, extracts of Coleus, extracts of Tephrosia, anti-stretch mark agents, in particular extracts of horse chestnut and escine, agents which protect or which improve microcirculation, in particular bioflavonoids of Ginkgo Biloba, and solar filters, in particular titanium oxides, Parsol MCX and filters of plant origin.

**20.** Use according to one of claims 1 to 19, **characterised in that** said composition further contains at least one other active principle selected from the group constituted by anti-dandruff agents, such as extracts of Arctium lappa, chloroxylenol, resorcinol or zinc pyrithione, anti-seborrhoea agents such as a 5α-reductase inhibitor, in particular an extract of Pygeum africanum, and agents which stimulate blood microcirculation, such as cepharanthine and methyl nicotinate.

**21.** A method of cosmetic treatment intended to obtain an improvement of the junction between the dermis and the epidermis by a reinforcement of the dermo-epidermal junction, or to stimulate the formation of collagen VII, **characterised in that** it consists in applying a cosmetically effective amount of an extract of the plant *Potentilla erecta*, said extract being contained in a composition comprising a cosmetically acceptable excipient.

**22.** The method of cosmetic treatment according to claim 21, **characterised in that** it consists in applying a cosmetically effective amount of an extract of the plant *Potentilla erecta* to obtain a toning of the skin, to prevent or to delay the appearance of signs of skin ageing, to delay the appearance of wrinkles or to decrease their depth, said extract being contained in a composition comprising a cosmetically acceptable excipient.